# EUROPÄISCHE PATENTSCHRIFT

(11) **EP 0 535 430 B1**
(45) Veröffentlichungstag und Bekanntmachung des Hinweises auf die Patenterteilung: **01.05.1996**
(21) Anmeldenummer: 92115655.0
(22) Anmeldetag: 14.09.1992
(51) Int. Cl.: C07C 29/76, C07C 41/09, C07C 41/34, C07C 41/36

(54) **Verfahren zur Herstellung von Dimethylether**
Process for the preparation of dimethylether
Procédé pour la préparation de l'éther diméthylique

(30) Priorität: 04.10.1991 DE 4132993
(43) Veröffentlichungstag der Anmeldung: 07.04.1993
(73) Patentinhaber: RWE-DEA Aktiengesellschaft für Mineraloel und Chemie, D-22204 Hamburg (DE)
(72) Erfinder: Hammer, Hartmut, Dr., W-5000 Köln 51 (DE)
(74) Vertreter: Schupfner, Gerhard D.

(56) Entgegenhaltungen:
- EP-A- 0 061 057
- EP-A- 0 460 311
- DD-A- 270 901
- FR-A- 2 403 323
- CHEMICAL ABSTRACTS, vol. 95, no. 7, 1981, Columbus, Ohio, US; abstract no. 61482, Seite 61496;
- DATABASE WPI Section Ch, Week 7030, Derwent Publications Ltd., London, GB; Class E17, AN 70-52648R & SU-A-253 040

## Beschreibung

Die vorliegende Erfindung bezieht sich auf ein Verfahren zur Geruchsverbesserung von aus Methanol hergestelltem Dimethylether und insbesondere auf ein Verfahren zur Herstellung von Dimethylether ohne Fremdgeruch durch Dehydratisierung von Methanol und anschließende destillative Aufarbeitung des rohen Dimethylethers.

Es ist allgemein bekannt, daß die bisher als Treibmittel eingesetzten Fluorchlorkohlenwasserstoffe eine beeinträchtigende Wirkung auf die Ozonschicht in der Stratosphäre haben, da sie aufgrund ihrer chemischen Stabilität genügend langlebig sind, um in diesen Bereich vorzudringen, sich dort unter Einfluß des Sonnenlichts mit dem Ozon umsetzen und damit den zur Herabsetzung von UV-Einstrahlung wichtigen Ozon-Schutzgürtel zerstören.

Dimethylether verursacht einen solchen Schaden nicht. Der Ether ist wasserlöslich und photochemisch vergleichsweise reaktiv, so daß er durch Umsetzungen zerstört wird, bevor er in die Ozonschicht gelangt.

Bekanntlich enthalten Spraydosen den eigentlichen Wirkstoff wie beispielsweise Deodorants, Parfüms, Insektenbekämpfungsmittel, Farblacke mit einem Lösungsmittel, bevorzugt Ethanol, und das Treibmittel.

Dimethylether macht es aufgrund seiner Polarität möglich, auch Wasser bzw. Wasser-Alkoholgemische als Lösungsmittel zu verwenden, da er in Wasser löslich ist. Andererseits ist er auch als Treibmittel einsetzbar, wenn unpolare Lösungsmittel, wie z.B. Kohlenwasserstoffe verwendet werden. In beiden Fällen werden homogene Gemische ohne Phasentrennung gebildet, so daß das Treibmittel nicht vorzeitig aus der Dose entweicht, sondern eine vollständige Entnahme des im Lösungsmittel gelösten Wirkstoffs gewährleistet ist. Dimethylether besitzt demgemäß hervorragende Eigenschaften als Treibmittel. Dimethylether hat daher während der letzten Jahre zunehmend an Bedeutung als Treibmittel in Spraydosen gewonnen.

Voraussetzung für die Verwendung des Dimethylethers als Treibmittel, vor allem im Haushalts- und Körperpflegebereich, ist jedoch, daß dieser weitgehend geruchsfrei ist.

Dimethylether (DME) wird aus Reinmethanol durch Dehydratisierung gewonnen. Reinmethanol besteht zwar zu 99,9 Gew.-%. aus Methanol, enthält jedoch zahlreiche Verunreinigungen im ppm-Bereich, und es entstehen bei der Umsetzung des Methanols geruchsaktive Nebenprodukte.

Typische Verunreinigungen sind Dimethylamin (Kp. 6,9 °C), Dimethylsulfid (Kp. 37,3 °C), Methylmercaptan (Kp. 5,8 °C), Formaldehyd (Kp. -21 °C), Formaldehyddimethylacetal (Kp. 45,5 °C), Trimethylamin, Verbindungen von Mono- und Dimethylamin mit Formaldehyd, Ameisensäure, Ameisensäureamide, Ameisensäuremethylester (Kp. 31,5 °C), Essigsäure, Essigsäureamide, Essigsäuremethylester (Kp. 56,95 °C), Kohlenwasserstoffe und andere in Spuren vorliegende Geruchsträger.

Da die Siedepunkte eines Teils der Verunreinigungen nahe zusammenliegen und diese zudem zahlreiche azeotrope Gemische bilden, ist es außerordentlich schwierig, eine insbesonders geruchsmäßig ausreichende destillative Abtrennung der Verunreinigungen zu erreichen, zumal die Verunreinigungen in der Regel nur in Spuren vorliegen.

Die Dehydratisierung des Methanols erfolgt durch heterogene Katalyse, vorzugsweise bei etwa 250 bis 400 °C und einem Druck von etwa 8 - 15 bar. Als Katalysatoren können Al₂O₃, SiO₂, Aluminiumsilikat und bevorzugt γ-Aluminiumoxid eingesetzt werden. Die Aufarbeitung des dabei gebildeten Reaktionsgemischs, das im wesentlichen aus Methanol, Wasser und Dimethylether besteht,erfolgt destillativ, siehe z.B. DD-Patent 270 901 A1.

Während der letzten Jahre wurden verschiedene Verfahren zur Gewinnung eines weitgehend geruchsfreien Dimethylethers beschrieben. So wird in der EP 0 124 078-A ein Verfahren beschrieben, wonach einer ersten, unter Druck stehenden Kolonne Dimethylether im Seitenabzug entnommen wird, während aus einer zweiten, unter niedrigerem Druck als die erste arbeitenden Kolonne über Kopf die zwischen Dimethylether und Methanol siedenden Verunreinigungen abgezogen werden. Methanol wird der zweiten Kolonne im Seitenabzug entnommen. Obgleich mit diesem Verfahren ein reinerer Dimethylether gewonnen werden kann als bislang möglich, muß nicht nur die erste, sondern auch die zweite Destillationskolonne mit hoher Bodenzahl ausgestattet sein, so daß sowohl erhebliche Investitionskosten als auch insbesondere erhebliche Betriebskosten entstehen. Ferner kann nicht völlig ausgeschlossen werden, daß zwischen Dimethylether und Methanol siedende Verunreinigungen nach Anreicherung in der ersten Kolonne mit dem Dimethylether abgezogen werden, statt vollständig in die zweite Kolonne überführt zu werden. Das Dimethylether ist dann nicht frei von Fremdgeruch.

In der EP-0 270 852-A2 wird ein Verfahren beschrieben, gemäß dem Dimethylether aus Methanol in Gegenwart eines γ-Al₂O₃ -Katalysators mit sehr geringem SiO₂ -Gehalt erzeugt und daraus in einem destillativen Reinigungsverfahren ein hochreiner, weitgehend geruchsfreier Dimethylether gewonnen wird, wobei die zwischen Dimethylether und Methanol siedenden Verunreinigungen in der gleichen Kolonne an bestimmten Böden im Seitenabzug abgetrennt werden. Eine Weiterentwicklung dieses Verfahrens wird in der europäischen Patentanmeldung EP- 0 340 324-A2 offenbart.

Unter störungsfreien, optimalen Bedingungen ist es möglich, einen als Treibmittel geeigneten Dimethylether nach diesen Verfahren zu gewinnen. Es ist dem Fachmann jedoch bekannt, daß die Trennung und Reinigung von Gemischen bzw. bestimmter Gemischanteile durch fraktionierte Destillation nur dann möglich ist, wenn sich die Destillationskolonne im Gleichgewicht befindet. Bei Störungen, Änderungen der Fahrweise, z.B. durch Änderung der Belastung, An- und Abfahren oder bei unterschiedlichen Mengen an eingetragenen Verunreinigungen ist jedoch das Gleichgewicht gestört, so daß dann der erforderliche Reinigungsgrad nicht mehr erreicht wird und ein Dimethylether erzeugt wird, der als Treibmittel nicht mehr geeignet ist.

In EP-A1-0 460 311, einem älteren Recht (Veröffentlichungstag = 11/12/91), ist ein Verfahren zur Entfernung von Geruchsverunreinigungen aus Dimethylether beschrieben. Dort sind mit Säure aktivierte Tone der Montmorillonit-Gruppe als wirksam zur Geruchsverbesserung des Dimethylethers herausgestellt, während eine Vielzahl typischer Absorbentien, darunter Ionenaustauscherharze und Tonminerale, für unwirksam befunden wurden.

Die Bestimmungen geruchsbelästigender Substanzen erfolgt noch immer überwiegend empirisch, wobei direkte sensorische Messungen durch ein geübtes Team erfolgen. So wurde beispielsweise die Belästigungsschwelle "BS" für H₂S durch ein Testkollektiv von 150 Personen auf 45 µg/m³ ermittelt (Schriftenreihe der Landesanstalt für Immissionsschutz des Landes Nordrhein-Westfalen, Heft 49 (1979) S. 77).

In den Fällen, in denen die Geruchsschwelle im durch Instrumente messbaren Bereich liegt, können Geruchsschwellen bzw. Belästigungsschwellen auch durch Messmethoden wie Gaschromatographie, elektrische Leitfähigkeit, Photometrie oder Fluoreszenzmessung bestimmt werden ("Erdöl und Kohle-Erdgas-Petrochemie", Bd. 32, Heft 2, Febr. 1975, S. 86).

Die Geruchsbestimmungen im Falle der vorliegenden Erfindung beruhen in erster Linie auf der sensorischen Methode.

Bei der Herstellung von insbesondere zum Einsatz als Treibmittel bestimmtem Dimethylether bestand daher die Aufgabe, Verunreinigungen aus dem Dimethylether soweit zu entfernen, daß dieser weitestgehend frei von fremden Geruchsträgern ist.

Die gestellte Aufgabe wird erfindungsgemäß dadurch gelöst, daß
(a) der Dimethylether-Produktstrom vor der destillativen Aufarbeitung gereinigt wird und/oder
(b) der Dimethylether-Produktstrom während der destillativen Aufarbeitung gereinigt wird, indem von einem Boden, der eine an Verunreinigungen reiche Fraktion des Dimethylethers enthält, oder im Bereich eines solchen Bodens der Destillationskolonne der Dimethylether abgezogen wird , diese Fraktion gereinigt wird und zum größeren Teil zur Kolonne zurückgeführt wird und zum kleineren Teil ausgekreist werden kann und/oder
(c) der Dimethylether nach der destillativen Aufarbeitung gereinigt wird,
wobei die Reinigung durch die Behandlung mit sauren Ionenaustauschern, ausgenommen saure Tone der Montmorillonit-Gruppe erfolgt.

Das als Einsatzmaterial vorgesehene Methanol vor Einsatz in die Synthese zur Herstellung des Dimethylethers kann zusätzlich auch mit sauren Ionenaustauschern behandelt werden.

Es wurde nämlich überraschenderweise festgestellt, daß Dimethylether ohne Fremdgeruch auch in den oben geschilderten Problemfällen durch dieses Verfahren erhalten werden kann.

Bevorzugt werden Kationenaustauscher auf Basis von vernetzten Styrolpolymerisaten eingesetzt. Es können jedoch auch mit Erfolg saure bzw. mit Säure behandelte Zeolithe eingesetzt und das Methanol-Einsatzmaterial zur Herstellung von Dimethylether bzw. der Verunreinigungen enthaltenden Dimethylether-Produktstrom über diese Materialien geleitet werden.

Ausführungsformen der vorliegenden Erfindung sind in den beigefügten Zeichnungen dargestellt und im folgenden näher beschrieben. Sie zeigen die folgenden Verfahrensschemata:
- Fig.1: Ionenaustauscher-Behälter zur Behandlung des Verunreinigungen enthaltenden Dimethylethers, installiert im diesbezüglichen Seitenabzug der Destillationskolonne oder sowohl oberhalb als auch unterhalb dieses Seitenabzugs.
- Fig.2: Ionenaustauscher-Behälter zur Behandlung des Dimethylether-Produktstroms, installiert in der Zuführung des Produktstroms zur Destillationskolonne.
- Fig.3: Ionenaustauscher-Behälter zur Behandlung des reinen Dimethylethers, installiert im diesbezüglichen Seitenabzug der Destillationskolonne.
- Fig.4: Ionenaustauscher-Behälter zur Behandlung des Methanol-Einsatzmaterials, installiert in der Zuführung zum Synthesereaktor.

Obgleich, wie bereits oben ausgeführt, das zur Herstellung des Dimethylethers verwendete Reinmethanol eine Vielzahl von Verunreinigungen enthält, die nach der Herstellung des Dimethylethers entweder chemisch unverändert oder als Folge des Kontaktes mit dem Dehydratisierungskatalysator chemisch verändert im rohen Dimethylether vorliegen, und obgleich die Mengen, in denen diese Verunreinigungen vorliegen, überwiegend mit üblichen Analysemethoden nicht nachweisbar sind, hat die Anmelderin überraschend gefunden, daß durch Überleiten des "Reinmethanols" oder von Dimethyletherfraktionen über die sauren Ionenaustauscher auch in den Fällen, in denen die Dimethylether-Destillationskolonne nicht im Gleichgewicht ist, ein Dimethylether erhalten wird, der frei von Fremdgeruch ist. Demgemäß genügt es, aus der Vielzahl von Verunreinigungen unterschiedlicher chemischer Struktur, basische Komponenten zu entfernen, um in jedem Betriebszustand ein einwandfreies Produkt zu erhalten, wobei jedoch zusätzliche Umsetzungen an den sauren Materialien stattfinden können, wie Esterbildung, Amidbildung und ähnliche Reaktionen. Darüber hinaus erlaubt das erfindungsgemäße Verfahren grundsätzlich, mit einer DME-Destillationskolonne zu arbeiten, die nur eine geringere Bodenzahl aufweist. Damit ist es möglich, wirtschaftlicher und mit geringeren Investitionen einen geruchsfreien Dimethylether herzustellen. Auch das Rückflußverhältnis kann gegenüber der Fahrweise ohne Behandlung mit sauren Ionenaustauschern in der Destillationskolonne zur Gewinnung des reinen Dimethylethers verringert werden.

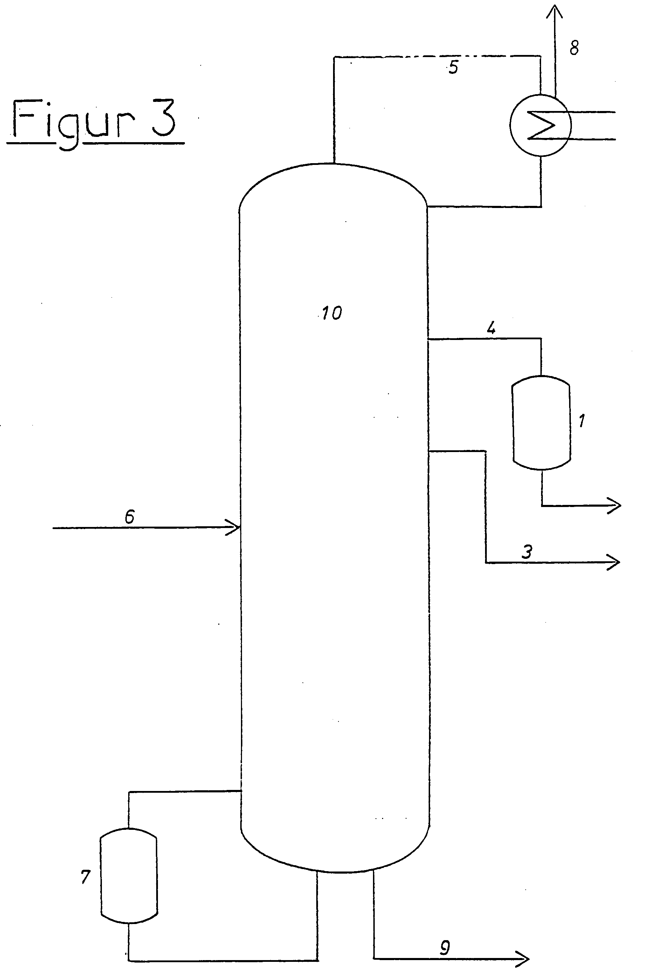

Als saure Ionenaustauscher können grundsätzlich alle auf dem Markt befindlichen Produkte eingesetzt werden, jedoch ist insbesondere im Falle der Fahrweise gemäß Figur 3 zu beachten, daß der Ionenaustauscher in Dimethylether völlig unlöslich ist, um den reinen Dimethylether nicht mit gelöstem Ionenaustauscher zu verunreinigen. Beispielsweise sind folgende Ionenaustauscher genannt:
Lewatite, wie z.B. Lewatit S 100 (Beladekapazität 1-1,2 Val/l), Lewatit SP 112 (Beladekapazität 0,8-1 Val/l), Lewatit SP 120 (Beladekapazität 0,6-0,8 Val/l); Amberlyste wie z.B. Amberlyst 15 (Beladekapazität ca. 1 Val/l) und zahlreiche andere Typen, die dem Fachmann wohl bekannt sind. Lewatit und Amberlyst sind eingetragene Warenzeichen.

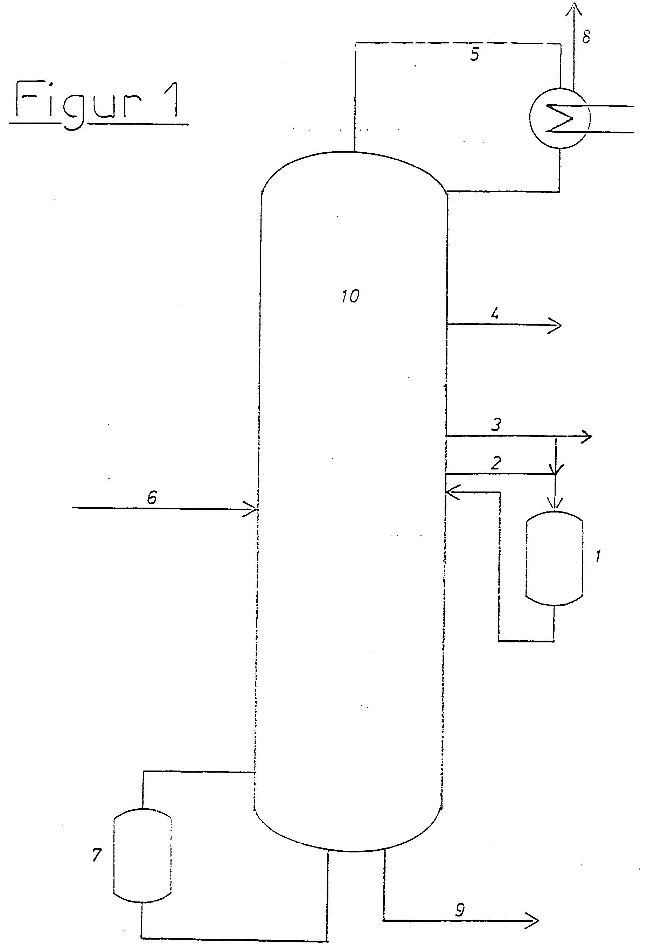

Bei den Fahrweisen gemäß den Figuren 1, 2 und 4 ist völlige Unlöslichkeit der sauren Ionenaustauscher von Vorteil, jedoch nicht zwingend erforderlich.

Der Ionenaustauscher ist in fester Form im Behälter bevorzugt als Festbett angeordnet. Ansonsten ist die Anordnung des Ionenaustauschers im Behälter Stand der Technik.

Es können auch zwei oder mehr Behälter mit sauren Ionenaustauschern, vorzugsweise in Parallelschaltung, zusammengeschaltet werden. Eine solche Schaltung ist ebenfalls Stand der Technik.

Mit Hilfe der Figuren wird im folgenden das erfindungsgemäße Verfahren näher erläutert.

In Figur 1 ist der Ionenaustauscher im Behälter 1 als Festbett angeordnet. Über Leitung 4 wird eine Fraktion reinen Dimethylethers (Fraktion 1) aus der Destillationskolonne 10 abgezogen. Über Leitung 3 wird eine Fraktion aus mit Verunreinigungen angereichertem Dimethylether (Fraktion 2) abgezogen. Ein Strom aus Leitung 3 oder Leitung 2 wird durch Behälter 1 in die Destillationskolonne zurückgeführt. Dieser Strom kann auch von unten nach oben über den Ionenaustauscher geleitet werden. Als Ionenaustauscher wurde Lewatit SP 112 eingesetzt.

Die Menge des durch den Behälter geführten Stroms beträgt im allgemeinen 1-2 Gew.-% des in die Destillationskolonne eingesetzten rohen Dimethylethers.

Zweckmäßigerweise wird der Strom, der über den Ionenaustauscher geleitet wird, an einem Boden abgezogen, der sich nahe dem Boden befindet, aus dem Fraktion 2 abgezogen wird, da in diesem Bodenbereich die Konzentration an Verunreinigungen relativ hoch ist. Vorzugsweise wird der Strom an dem Boden selbst abgenommen, an dem Fraktion 2 abgezogen wird. Der Boden, an dem Ether zur Reinigung mit Ionenaustauscher abgezogen wird, kann sich somit unter oder über dem Boden befinden oder gleich sein, an dem die Fraktion 2 abgezogen wird, die mit Verunreinigungen angereichert ist. Wird über Leitung 3 eine gleichgroße Menge wie über Leitung 4 der Kolonne zunächst entnommen, und nach Ausschleusung von 1-2 % davon im wesentlichen über den Ionenaustauscher zurückgefahren, so kann die Bodenzahl zwischen Leitung 6 und Leitung 4 um etwa 60% verringert werden. Leitung 6 stellt die Zuführung für den rohen Dimethylether dar, Leitung 5 die Abzugsleitung am Kopf der Kolonne und Leitung 8 die Entnahme von Leichtsiedern, die niedriger sieden als Dimethylether. Leitung 7 stellt den Reboiler dar und Leitung 9 den Sumpfabzug.

Mit 2000 kg saurem Ionenaustauscher wurde bei einer Produktion von 10 m³/h an reinem Dimethylether über 8.000 Stunden (1 Jahr) ohne Regeneration des Ionenaustauschers Dimethylether in geruchsfreier Qualität erzeugt. Obgleich Schwankungen in der Belastung der Destillationskolonne und An- und Abfahrvorgänge erfolgten, konnte der reine Dimethylether in nur einem Vorratstank mit gleichbleibend guter, geruchsfreier Qualität gesammelt werden. Dies traf auch zu für die im folgenden beschriebenen Fahrweisen.

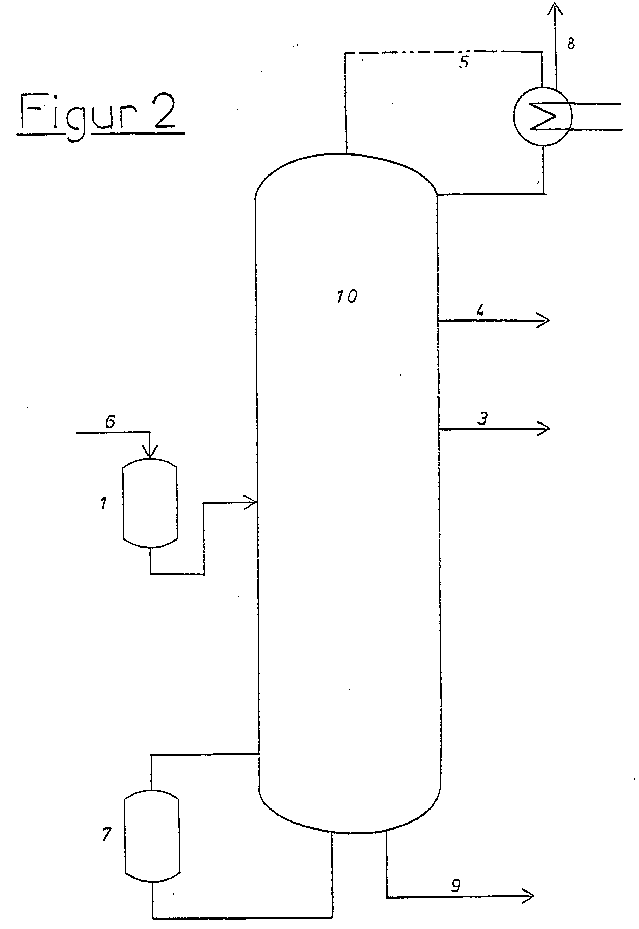

In Figur 2 befindet sich der Ionenaustauscher-Behälter 1 in der Zuführungsleitung bzw. bei mehreren, in mindestens einer Zuführungsleitung 6 für rohen Dimethylether. Als Ionenaustauscher wurde Amberlyst 15 eingesetzt.

In Destillationskolonne 10 ist wiederum 7 der Reboiler, 9 die Leitung zur Entnahme des Kolonnensumpfes, 5 die Leitung zur Kondensation und 8 die Leitung zur Entnahme der Leichtsieder.

Über Leitung 4 wird reiner Dimethylether entnommen. Über Leitung 3 wird eine mit Verunreinigungen angereicherte Fraktion entnommen.

Bei dieser Fahrweise läßt sich mit 4000 kg saurem Ionenaustauscher bei einer Produktion von 10 m³/h an reinem Dimethylether über 8.000 Stunden (1 Jahr) ohne Regeneration des Ionenaustauschers Dimethylether in geruchsfreier Qualität erzeugen. Trotz unterschiedlicher Belastung und An- und Abfahrvorgängen wurde einwandfreier Dimethylether erhalten. Die über Leitung 3 abgezogene Menge konnte auf 0,1-0,2 Gew.-% des eingesetzten rohen Dimethylethers herabgesetzt werden.

In Figur 3 ist eine Fahrweise dargestellt, bei der der Ionenaustauscher-Behälter 1 in der Leitung 4 für reinen Dimethylether installiert ist. Als Ionenaustauscher wurde Lewatit SP 120 eingesetzt.

Die sonstigen Apparateangaben sind unverändert. Ein Versuch für diese Fahrweise ergab, daß mit 200 kg saurem Ionenaustauscher bei einer Produktion von 10 m³/h reinem Dimethylether über 8.000 Stunden (1 Jahr) ohne Regeneration des Ionenaustauschers geruchsfreie Dimethyletherqualität erhalten wurde.

Bei Einsatz von 2000 kg saurem Ionenaustauscher konnte die über Leitung 3 abgezogene Menge auf 0,1-0,2 Gew.-% des eingesetzten rohen Dimethylethers herabgesetzt werden, bei entsprechend verbesserter Ausbeute an reinem Dimethylether.

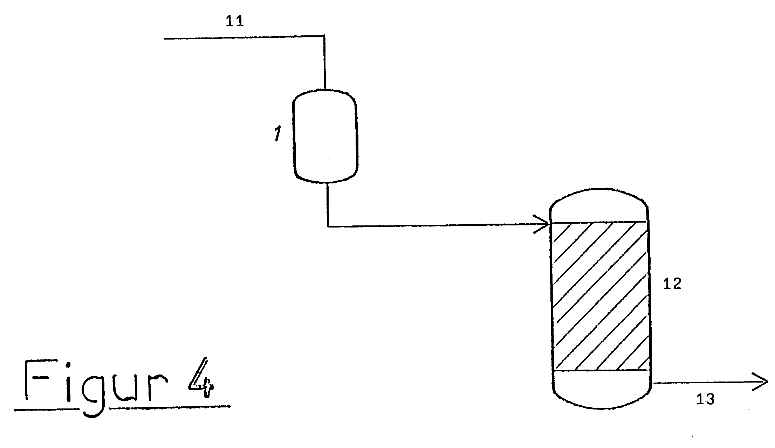

In Figur 4 ist eine Fahrweise dargestellt, bei der sich der Ionenaustauscher-Behälter 1 in der für die Zuführung von Reinmethanol zum Dimethylether-Synthesereaktor 12 vorgesehene Leitung 11 installiert ist. Durch Leitung 13 wird der rohe Dimethylether abgeführt.

Als Ionenaustauscher wurde Lewatit S 100 eingesetzt. Mit 4.200 kg Ionenaustauscher wurde bei einem Durchsatz von 10 m³/h Reinmethanol während 8.000 Stunden (1 Jahr) Fahrzeit ohne Regeneration des Ionenaustauschers eine geruchsfreie Dimethyletherqualität erhalten.

Obgleich die Menge an Ionenaustauscher gegenüber den Fahrweisen nach den Figuren 1 und 3 erhöht werden muß, wird auch in diesem Fall ein einwandfreies Produkt erhalten. Die über Leitung 3 abgezogene Menge konnte wie bei den Fahrweisen der Figuren 2 und 3 herabgesetzt werden.

Grundsätzlich kann ein Bett mit sauren Ionenaustauschern auch in der Synthesegaszuführung zum Synthesereaktor zur Herstellung von Methanol installiert werden, also noch eine verfahrensstufe früher als bei der Fahrweise gemäß Figur 4.

Diese Untersuchungen machen deutlich, daß das erfindungsgemäße Verfahren zu ganz erheblichen technischen und wirtschaftlichen Vorteilen führt.

## Patentansprüche

1. Verfahren zur Herstellung von Dimethylether ohne Fremdgeruch durch Dehydratisierung von Methanol und anschließende Destillation des rohen Dimethylethers, dadurch gekennzeichnet, daß
(a) der Dimethylether-Produktstrom vor der destillativen Aufarbeitung gereinigt wird und/oder
(b) der Dimethylether-Produktstrom während der destillativen Aufarbeitung gereinigt wird, indem von einem Boden, der eine an Verunreinigungen reiche Fraktion des Dimethylethers enthält, oder im Bereich eines solchen Bodens der Destillationskolonne der Dimethylether abgezogen wird , diese Fraktion gereinigt wird und zum größeren Teil zur Kolonne zurückgeführt wird und zum kleineren Teil ausgekreist werden kann und/oder
(c) der Dimethylether nach der destillativen Aufarbeitung gereinigt wird,
wobei die Reinigung durch die Behandlung mit sauren Ionenaustauschern, ausgenommen saure Tone der Montmorillonit-Gruppe erfolgt.

2. Verfahren nach Anspruch 1 dadurch gekennzeichnet, daß der Methanol Eduktstrom vor der Umsetzung mit sauren Ionenaustauschern behandelt wird.

## Claims

1. A process for producing dimethyl ether which is free from any foreign odour by dehydration of methanol and subsequent purifying distillation of the crude dimethyl ether **characterized by**
(a) purifying the dimethyl ether product stream prior to distillation and/or
(b) purifying the dimethyl ether product stream during distillation by withdrawing the dimethyl ether from a tray carrying a highly contaminated fraction of the dimethyl ether or in the area of any such tray of the distillation column, purifying said fraction and returning the greater part to the column, while the smaller part may be discarded and/or
(c) purifying the dimethyl ether after distillation,
wherein the purification is achieved by treatment with acidic ion-exchange resins, with the exception of acidic clays of the montmorillonite group.

2. A process according to claim **1** wherein the methanol feedstock stream is treated with acidic ion-ex-change resins prior to conversion.

## Revendications

1. Procédé de production d'éther de diméthyle sans odeur étrangère par déshydratation de méthanol suivie de la distillation de l'éther de diméthyle brut, caractérisé en ce que
(a) le courant de production d'éther de diméthyle est purifié avant le traitement de distillation et/ou
(b) le courant de production d'éther de diméthyle est purifié pendant le traitement de distillation, par le fait que l'éther de diméthyle est prélevé au niveau d'un plateau qui contient une fraction de l'éther de diméthyle riche en impuretés, ou dans la zone d'un tel plateau de la colonne de distillation, cette fraction est purifiée et recyclée en majeure partie à la colonne et peut être déchargée en moindre partie du circuit et/ou
(c) l'éther de diméthyle est purifié après le traitement par distillation,
la purification étant effectuée par traitement avec des échangeurs d'ions acides, excepté des argiles acides du groupe de la montmorillonite.

2. Procédé suivant la revendication 1, caractérisé en ce que le courant méthanolique de départ est traité avec des échangeurs d'ions acides avant la réaction.
